# EUROPEAN PATENT APPLICATION

(11) **EP 2 777 730 A1**
(43) Date of publication of application: **17.09.2014**
(21) Application number: 13159119.0
(22) Date of filing: 14.03.2013
(51) Int. Cl.: A61M 5/168, A61M 5/14

(54) **Drug infusion system comprising occlusion detector**

(71) Applicant: IN. Medica, d.o.o., 8310 Sentjernej (SI)
(72) Inventor: Pavlin, Marko, 8000 Novo Mesto (SI)
(74) Representative: Ivancic, Bojan

(57) **Abstract**

The present invention refers to a medical drug infusion system for dosing relatively small quantities of a fluid, comprising a container (1) of the fluid to be dosed, a fluid conduit (2) connected to an infusion catheter or needle (3), a pumping means (4) to transport the fluid to be dosed via fluid conduit (2) to the patient, and a sensor unit (5) which monitors the pressure within said fluid conduit (2). The sensor unit (5) is arranged downstream of the pumping means (4) at a fork (6) from the fluid conduit (2), a valve (7) being arranged between the fluid conduit (2) and the sensor unit (5), said valve (7) enabling at the predetermined pressure, which is higher than the usual pressure in the fluid conduit (2) such as during an occlusion, flow of the fluid to be dosed from said conduit (2) into said sensor unit (5) where the fluid is optically or acoustically detected.

## Description

The present invention refers to a new dosing device, particularly to a dosing device used in the medicine in order to dose a relatively small quantities of a fluid, comprising a container of the fluid to be dosed, a fluid conduit between said container being fluidly connected with a fluid recipient, a pumping means to transport the fluid to be dosed via fluid conduit to the fluid recipient, and a sensor unit which controls the fluid flow via said fluid conduit.

Dosing devices of the aforementioned kind are well known. Thus, for instance, a dosing device is known comprising a sensor unit to control flow of a fluid to be dosed in a form of a pressure sensor arranged at the outlet of the pumping means. Such a sensor is attached both to a hydraulic part of the dosing device and an electronic part thereof. The dosing device is intended to be disposable, i.e for a single use only, whereas the pressure sensor is intended to be non-disposable, i.e. to be used several times, therefore, the connection between both, in particular the electronic connection between both is relatively complex and money consuming. On the other hand, a disposable dosing device is known where the entire device is disposed after the use thereof, including a sensor unit which controls a fluid flow via fluid conduit. Such a dosing device is particularly money consuming, since the sensor unit is to be disposed in spite of the fact that the sensor unit per se is not contaminated.

It is the object of the present invention to create a dosing device of the aforementioned kind, which remedies drawbacks of known dosing devices.

The object as set above is solved by characteristics set forth in a characterising clause of the claim 1.

The invention is further described in detail by way of non-limiting preferred embodiment, and with a reference to the accompanying drawing, where
- Fig. 1: shows a schematic view of a dosing device according to the invention, and
- Fig. 2: shows a schematic view of a sensor unit which controls a fluid flow via a fluid conduit.

A dosing device according to the invention comprises a container 1 of a fluid to be dosed, a fluid conduit 2 between said container 1 and, for instance, a syringe 3 through which the fluid to be dosed is supplied to the fluid recipient, a pumping means 4 forcing the fluid to be dosed via a fluid conduit 2 to the fluid recipient, and a sensor unit 5 which controls the fluid flow via the fluid conduit 2. Said sensor unit 5 is arranged downstream the pumping means 4 at the fork 6 of the fluid conduit 2, a blocking means 7 arranged between said fluid conduit 2 and said sensor unit 5 that regulates, directs or controls the flow of said fluid. Said blocking means 7 is of the kind to allows for, at the predetermined pressure, the flow of the fluid to be dosed from said conduit 2 into said sensor unit 5.

Said sensor unit 5 comprises a fluid chamber 8 and a sensor 9. Said fluid chamber 8 is provided, preferably at the side averted from said fluid conduit 2, with specular planes 10, 11 arranged in the V-form against each other, preferably in a manner that the angle between said planes 10, 11 is a right angle. It is provided for, according to the present invention, that at least said planes 10, 11 of the chamber 8 are formed of a material being permeable for selected waves such as in form of electromagnetic waves of an arbitrary spectrum, i.e. visible or infra-red or ultraviolet spectrum for instance, and/or in form of mechanical waves of an arbitrary spectrum, such as ultrasound for instance, and similar. Externally to said chamber 8 and spaced there from is arranged a sensor 9 comprising an emitter 12 and a receiver 13. According to the present invention, said emitter 12 is selected as a source of said waves, a source of incident ray for instance, whereas said receiver 13 is selected as a receptor of said waves, a source of reflected ray for instance. Both the emitter 12 and the receiver 13 are capable of operating either continuously or intermittently or as a part of a closed regulation loop.

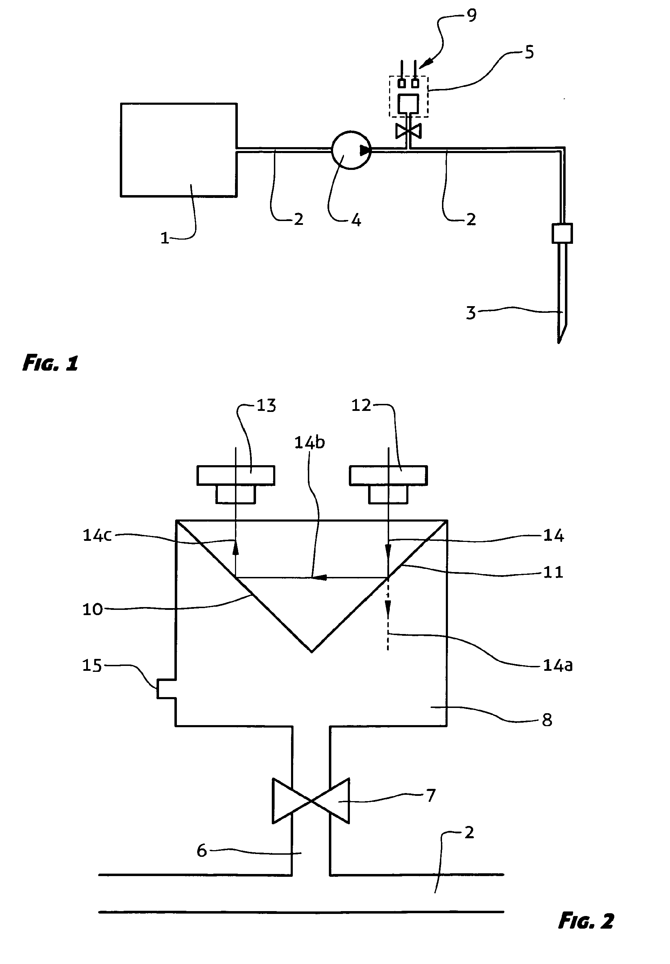

During usual regular operation said fluid chamber 8 is empty. It contains only air since said blocking means 7 prevents fluid to be dosed to enter the chamber 8 from the fluid conduit 2. Since the planes 10, 11 are permeable for selected waves and the chamber 8 contains only air, the refractive index *n* in this case amounts to approximately *n=1*. A ray 14 of an incident light for example emitted by said emitter 12 strikes the first plane 11 arranged in the area below said emitter 12, however, due to said refractive index said ray 14 propagates without reflection through said plane 11 as a ray 14a of a transmitted light, for example (cf. dashed line in Fig. 2).

When, however, the pressure of the fluid in the fluid conduit 2 increases due to any kind of an obstacle in the flow over the predetermined pressure to which said blocking means 7 is set, the latter unblocks the fluid flow via said fork 6 into the fluid chamber 8 which fills up with the fluid. The air is exhausted from the chamber 8 through a vent valve 15. As a result, the refractive index *n* is changed, in particular it increases to *n>1*. If said fluid to be dosed, is insulin for instance, said refractive index n amounts to approximately *n=1,3.* The ray 14 of the incident light for example, being emitted by said emitter 12 is reflected due to changed refractive index as a ray 14b of a light for example, from the first plane 11 to said second plane 10 arranged in the area below said receiver 13 of the reflected light for example, and is reflected from said second plane 10 as a ray 14c of said reflected light towards the receiver 13. The latter senses the arrival of the reflected light and communicates this information to a control unit not shown which triggers necessary steps.

Thus, the dosing device according to the present invention is intended to be a dosing device for a single use whereas the sensor 9 is entirely separated fro the dosing device and is intended for a multiple use.

While the foregoing written description enables one of ordinary skill to make and use what is considered presently to be the best mode thereof, those of ordinary skill will understand and appreciate the existence of variations, combinations, and equivalents of the specific exemplary embodiments and methods herein. The invention should therefore not be limited by the above described embodiments and methods, but by all embodiments and methods within the scope and spirit of the invention as claimed.

## Claims

1. A dosing device, particularly a dosing device used in the medicine in order to dose a relatively small quantities of a fluid, comprising a container (1) of the fluid to be dosed, fluid conduit (2) between said container (1) being fluidly connected with a fluid recipient, a pumping means (4) to transport the fluid to be dosed via fluid conduit (2) to the fluid recipient, and a sensor unit (5) which controls the fluid flow via said fluid conduit (2), ***characterized in that*** a sensor unit (5) is arranged downstream of a pumping means (4) at a fork (6) from a fluid conduit (2), a blocking means (7) being arranged between the fluid conduit (2) and the sensor unit (5), said blocking means (7) enables at the predetermined pressure, which is higher than the usual pressure in the fluid conduit (2), flow of the fluid to be dosed from said conduit (2) into said sensor unit (5).

2. A dosing device according to claim 1, ***characterized in that*** said sensor unit (5) comprises a fluid chamber (8) and sensor (9).

3. A dosing device according to claim 2, ***characterized in that*** said fluid chamber (8) is provided with planes (10, 11) arranged in the V-form against each other.

4. A dosing device according to claim 2, ***characterized in that*** the angle between said planes (10, 11) is preferably a right angle.

5. A dosing device according to claim 2, ***characterized in that*** said planes (10, 11) are preferably arranged at the side of the fluid chamber (8) averted from the fluid conduit (2).

6. A dosing device according to claims 1 to 5, ***characterized in that*** at least said planes (10, 11) of the chamber (8) are formed of material which is permeable for selected waves.

7. A dosing device according to claim 6, ***characterized in that*** said waves are selected from any part of electromagnetic spectrum, such as visible light, infra-red light, ultraviolet light, etc.

8. A dosing device according to claim 6, ***characterized in that*** said waves are selected from any part of mechanical waves spectrum, such as ultrasound, etc.

9. A dosing device according to claims 1 and 2, ***characterized in that*** the sensor (9) arranged externally to said chamber (8) and spaced from the latter comprises an emitter (12) and a receiver (13).

10. A dosing device according to claim 9, ***characterized in that*** said emitter (12) is selected as a source of said waves, whereas said receiver (13) is selected as a receptor of said waves.

11. A dosing device according to claims 1 to 10, ***characterized in that*** the emitter (12) and the receiver (13) are capable of operating continuously.

12. A dosing device according to claims 1 to 11, ***characterized in that*** the emitter (12) and the receiver (13) are capable of operating intermittently.
